# EUROPEAN PATENT APPLICATION

(11) **EP 2 392 640 A1**
(43) Date of publication of application: **07.12.2011**
(21) Application number: 09839069.3
(22) Date of filing: 30.01.2009
(51) Int. Cl.: C12M 1/00, C12M 3/00

(54) **METHOD AND MODULAR EQUIPMENT FOR THE PROCESSING AND STORAGE OF CELL CULTURES**

(71) Applicant: Mateu Sentamans, Emilio, 46610 Guadassuar (Valencia) (ES)
(72) Inventor: Mateu Sentamans, Emilio, 46610 Guadassuar (Valencia) (ES)
(74) Representative: Chanza Jordan, Dionisio
(86) International application number: PCT/ES2009/000051
(87) International publication number: WO 2010/086465

(57) **Abstract**

The invention relates to a method and modular equipment for the processing and storage of cell cultures. The invention includes a chain column of cells (6), in the form of a bucket conveyor, inside a bioreactor (1), moving continuously by means of a set of two rotors (8 and 9) which facilitates stable and uniform moisture/temperature conditions inside same. Each cell (7) contains one or more culture beds (12). The culture is supplied by means of a part (18) allowing injection and sterilisation using electromagnetic, gas or radiation means, which part is centrifuged by a controlled centrifugation mechanism in which trapping is activated by the centrifugal force (27). The invention can also include a microscopic module (28) which captures images and a telematic server/client configuration connected to the data processing unit and/or external units. It is possible to use one or more modular column parts (6), centrifuges (27) and microscopes (28).

## Description

### Technical Sector

The invention that is protected in this patent is a method and modular equipment for the processing and storage of cell cultures for the implementation of the culture, processes of diagnosis, testing and maintenance of cells and biological tissues.

It is, therefore, a method and equipment that allow the control and management of biological material for biotechnological applications in the field of medicine (molecular diagnosis and prognosis of diseases, cell therapy and tissue engineering, genetics, gene therapy and vaccines gene, regenerative medicine for the reconstruction of tissues and organs), pharmacology (drugs), food (GM food, fermented beverages, fortified or allergy, etc ...) very useful in basic and applied scientific research related to Genomics, Proteomics and Systems Biology, also called 'bioinformatics'.

In conclusion, this method and the cultivation equipment facilitate the diagnosis and conservation of biological material both scientific and industrial applications related to biotechnology in any technical field - energy, health, agriculture and forestry, livestock, food, etc... - and they are capable to perform more than four simultaneous experiments in each of the hundreds of available cell seeding.

### Background art

The use of culture culture, diagnostics and conservation of biological material is known and is extended with various techniques. However, these various techniques and procedures vary depending on both technical and its applications. To this aim, the present invention is achieved by an equipment of management and control over biological material advantages over those already known because of their functional and operational characteristics in order to its modularity and versatility of configurations depending on the features of their use.

Since the initial cultures and rudimentary biological material - cells and tissues - with pipettes in Petri dishes in 1877 to the present, various conventional techniques arise that we will discuss.

Tissue culture can be classified into two broad categories of techniques: organ culture and cell culture. The organ culture is the maintenance of small fragments of tissue or entire organs in vitro, while cell culture, involves the propagation of scattered cells both in suspension in monolayers on glass or plastic (M.REINA and CARME AULADELL, "Concepts Basic cell culture "/ University of Barcelona).

The first objective of the study was to achieve cell growth culture necessary and appropriate for cell growth. To do this, BURROWS (1910) used chicken plasma, later supplemented with embryonic extracts. CARREL and BURROWS got mammalian cell cultures, but nevertheless, they encountered problems of contamination of crops by the mere manipulation. CARRELL himself (1913) through its device called 'Carrel flask cell culture' was maintained for prolonged periods of time.

Develop means of necessary nutrients, and proper aseptic conditions were the main challenges in the beginning of tissue culture.

Currently, three types can be grouped in tissue culture: The culture of organs, primary explants, and cell culture.

The organ culture, maintains the characteristics of the tissue 'in vivo' but holds a limited spread of the tissue of origin, at most in the borders. The primary explants, however, favor the crop is produced in certain surfaces an explant or fragment of tissue where cells proliferate. Finally, cell culture, in which enzymatic or mechanical means - whether manual or automated - there is a cell disruption, facilitating their spread and cell mass culture for generations.

Cell culture characterized by the high spread of uniform and homogeneous populations, enabling their characterization, quantification, maintenance and replication samples.

Currently, to develop these techniques of cultivation, and in particular cell culture and tissue are already known and used various methods and devices. But they all have drawbacks operational and functional, especially for its ease in being contaminated zones in altered cell growth or environmental conditions and tissue cell proliferation, or low productivity. As always all of them involve some limitations compared to the invention is presented. Thus, they are known as devices, inventions relating to specific technical means.

By way of example, with the purpose of maintaining cell cultures U.S. Patent No. 4537860 MONSANTO COMPANY (1985) on a static or a crate portable cell cultures with tubes and the European Patent EP0046681 MONSANTO COMPANY (1982) which highlights the need for constant stirring of the crops, or also an incubator autonomous by PERSONAL DIAGNOSTIC as EP0182926 (1986).

They are also known technical combined, ie that perform various functions at once, such as U.S. Patent No. 5424209 KEARNEY G. (1995) on an automated cultivation and diagnosis, or the Spanish Patent ES2162081 AASTROM BIOSCIENCES (2001) on equipment and procedures for maintenance and growth of biological cells from the international patent W9609770US.

This patent, AASTROM BIOSCIENCES reflects a basic provision of means that allows automation and modularity in the culture, diagnosis and maintenance of biological material. Being designed for occasional use with a little industrialization.

Searching for automation control and management of cell cultures, is needed to increase the level of scientific and industrial efficiency at different applied fields or sectors.

Thus, U.S. Patent No. 6673595 of BIOCRYSTAL (2004) combines the functions of culture, diagnosis, and conservation. However, this inventive concept is limited by its own configuration storage due to cultivation beds in vertical towers, which are vulnerable to changes in temperature or environmental conditions inside the bioreactor, in particular, by stacking heights of the crops that can cause environmental states are not uniform or homogeneous, which may affect or thwart some of the cultured devices.In addition to its design, it makes available in modules for a process of efficient and rapid analysis of the state of cell cultures and biological tissues.

### Technical Problem

One of the main obstacles in the path of joining in a single machine or equipment multifunctional aspects - cell and tissue growth, characterization and conservation - lies in promoting and maintaining optimum condition and suitable growing conditions uniformly throughout the chamber where they are deposited for cultivation, and their characterization and conservation. Aside from being a closed chamber, it should generate a physical, chemical and biological stable and controllable in culture culture that are very sensitive and susceptible to variation, especially in relation to the humidity and temperature, which is why the machine is equipped with numerous sensors to control all these variables within the entire bioreactor.

Many of the crops should be made and maintained with constant agitation and movement continued at speed to get a crop bed matched the entire surface attached (plate, dish, suitcase bioreactor, etc ...). Getting a precise control of agitation , therefore becomes essential in many industrial trials or growth of biological material. This system complies perfectly with the cell culture scientific protocols adopted by the scientific community, in particular the constant agitation that is caused by a flipping.

Finally, to increase the number of cell cultures that promotes scientific and industrial performance, requires cost-effective and technical means on each crop, a fact that is justified by their ability to work and the possibility of adding additional modules.

### Technical Solution

The present invention brings together in one compact equipment to the operational culture, characterization, and maintenance, obtaining environmental stability and uniformity throughout the interior of the bioreactor, a controlled and constant agitation of the cultivated beds, and a manipulation of the beds accurately and easily cultivated. Also, the design equipment facilitates the modular arrangement of one or more pieces of chains of cells in culture beds by innumerable amount of them, paid off by the use of a single technical equipment and management control also enables and facilitates conducting a large number of experiments simultaneously.

This helps provide results and lighting applications where the invention is incorporated, which do not exist in the art already known.

### Advantageous Effects

The advantages can be highlighted in the present invention can include:
- The layout of the beds of culture (dishes, plates or flasks platelets ), where it attaches grouped in cells growing in cell format chains of beds, storage and possible fit for cultivation in lines parallel columns of chains within of an equipment monitoring and managing them, and therefore many crops. Without the need to provide all the room in a controlled atmosphere and the possible risk of injurious effects to the beds by the mechanical elements or electronic manipulation.
- The modular design facilitates both the integration equipment with aligned parallel columns of chains of cells in the beds of crops such as technical solutions for its cultivation, characterization and storage for each row or column (parts centrifuges, microscopes, etc ...)
- The configuration of chains of cells in culture beds get a constant agitation of the cultures through its continuous turning in a bioreactor with the same characteristics and environmental parameters - remarkably, humidity and temperature - which do not occur in stacked configurations or turrets.
- That same configuration chain beds crop cells, promotes a characterization or diagnosis of the crops, handling and storing the data with minimal impact, because it is a joint as a fixed wheel that rotates vertically for all functions. May well increase the number of modules or test equipment or processes.
- The guidance system includes a special buffer that prevents unwanted movements beds may suffer either vertical, horizontal or any kind of vibration. This contributes to better stability of the beds and prevents possible damage or injuries thereof.
- The same configuration on vertical work flow can attenuate or compensate for temperature gradients, so that total homogenization is achieved in the way heat is transferred to the beds.

### Description of Drawings

For a better understanding of the general characteristics mentioned above, are accompanied by several drawings to the present invention which is disclosed as specified below:

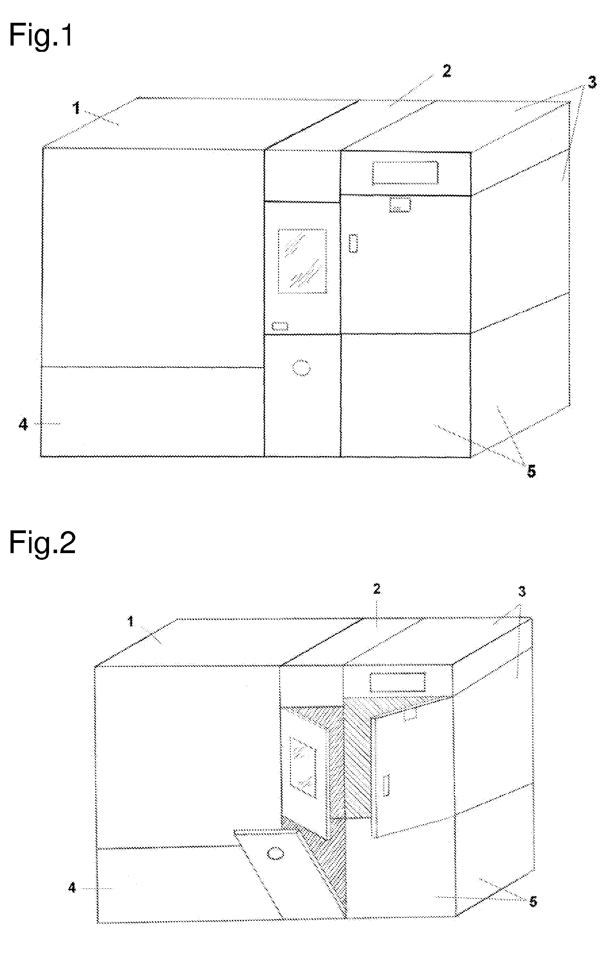

**Figure 1****:** Front view schematic of an equipment to grow, diagnosis and conservation of biological cells and tissues with a bioreactor (1), a load beds area and discharge of beds used (2), an area for culture supply and reagents (3), an area of pumping and centrifugation (4), and an area of computer data processing contains a microprocessor and storage of waste (5).
**Figure 2****:** Front view schematic of an equipment to grow, diagnosis and conservation of biological cells and tissues with a bioreactor (1), a load beds area and discharge of beds used (2), an area for culture supply and reagents (3), an area of pumping and centrifugation (4), and a processing area of computer data containing a microprocessor and storage of waste (5), with the gates open.

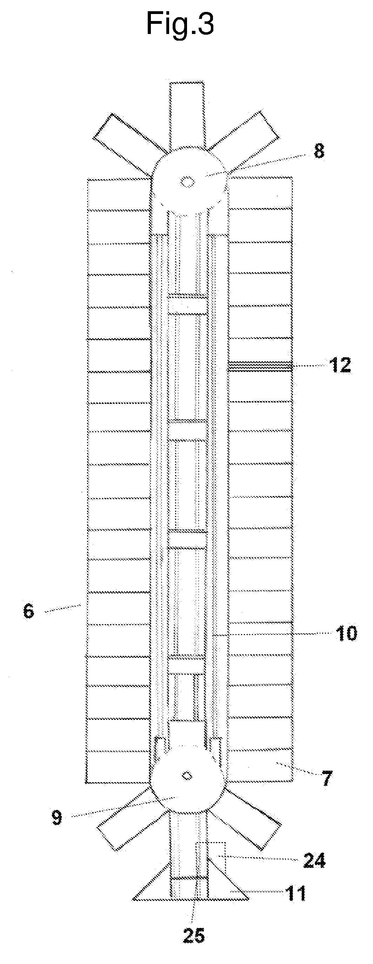

**Figure 3****:** Front view located inside the bioreactor (1) of a chain column of cells (6) with his foot column (11), link chain or strap cells (10), a cell (7) can contain one or more beds (12), a rotor-motor (8), a spinning rotor (9), a driving piece of the beds (24), and a lever of the beds (25).

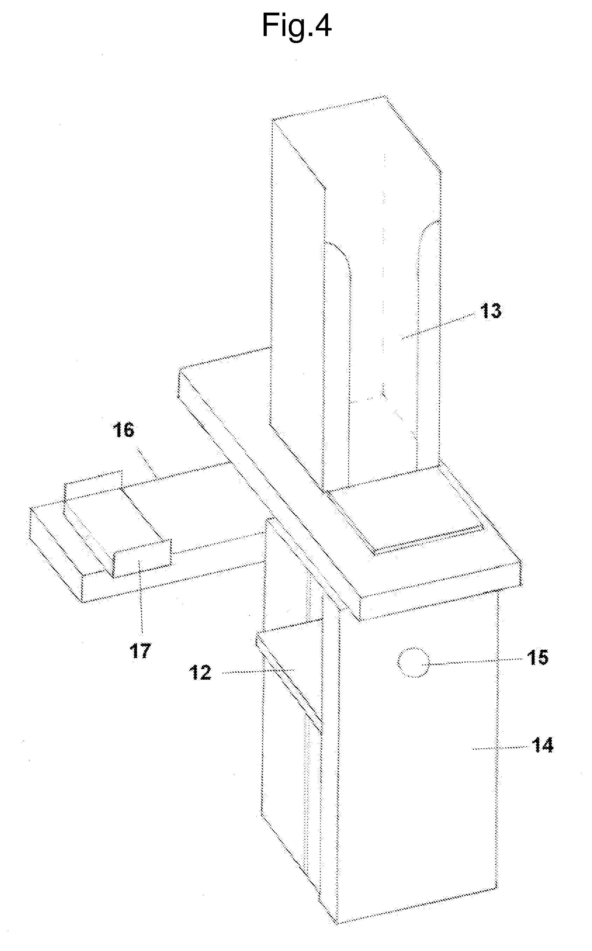

**Figure 4****:** Front view located in the load beds area and discharge of beds used (2), a bed (12), a tray of bedding to use (13), a transport guide (16 ), a transport support (17) that moves horizontally as maneuver to in or out respectively, an output tray (14), and an opening gap (15) of the tray.

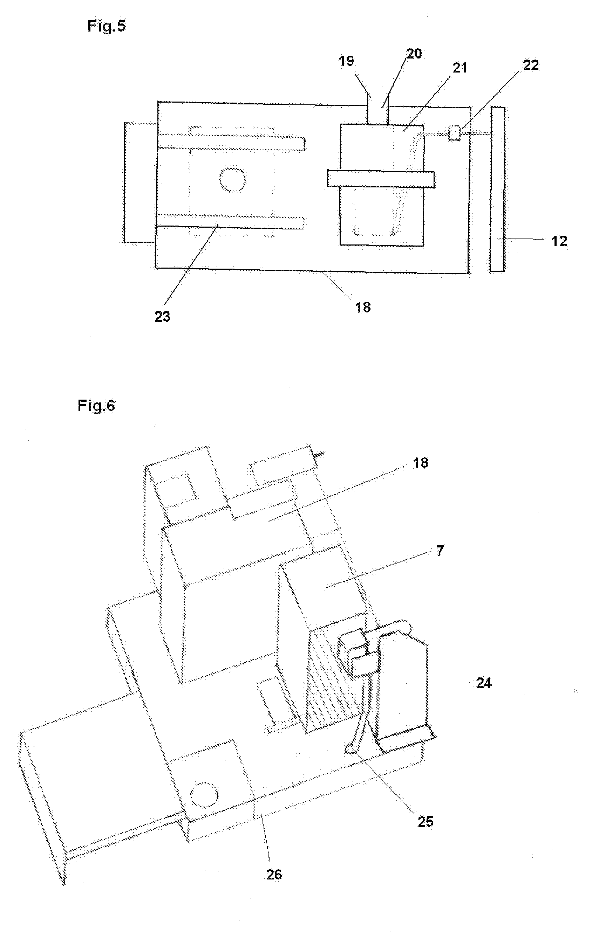

**Figure 5****:** Front view of a piece of injection and sterilization (18) located on the bottom inside the bioreactor (1), with a culture bed (12), a conduit for injection of compressed air (19), conduit injectable liquid (20), a pre-injection chamber (21), one face of the injector (22) and a sterilizing machine (23).
**Figure 6****:** Side view of a group located at the bottom inside the bioreactor (1), and composed the piece injection and sterilization (18), a cell belonging to the column in the chain of cells, a driving piece beds (24), a driving lever beds (25) and a mobile base of the piece driving beds (26) for horizontal scrolling.

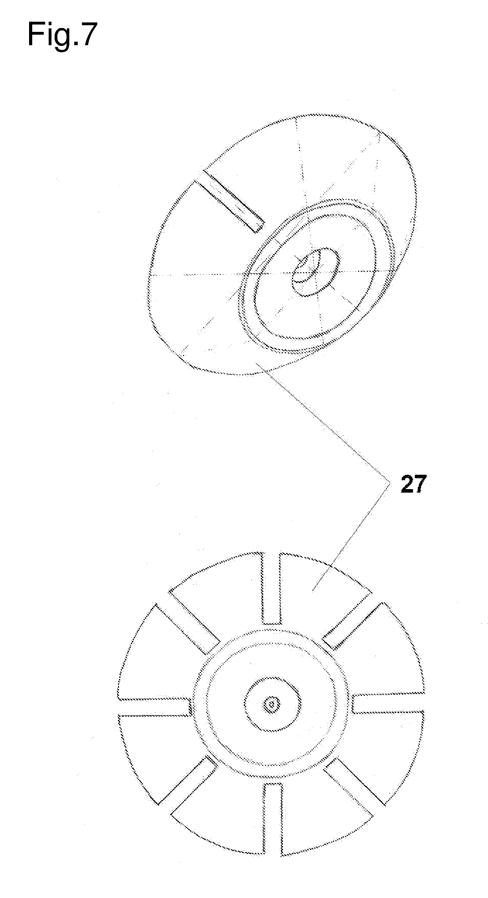

**Figure 7****:** View of a centrifuge (27) located on the bottom inside the bioreactor (1).

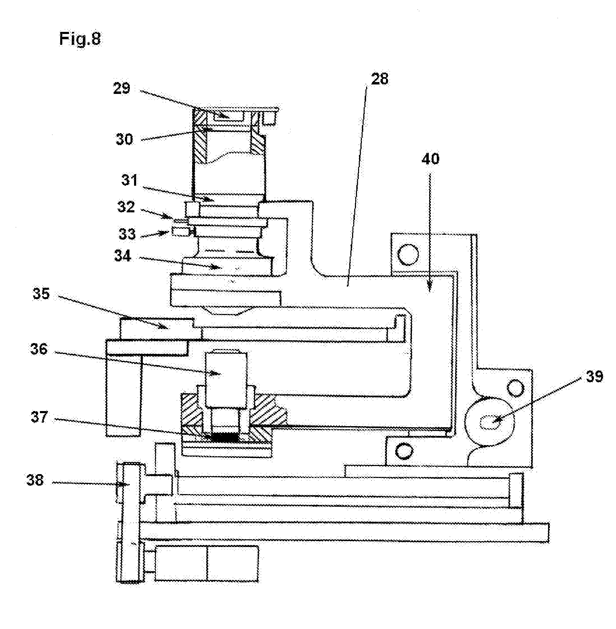

**Figure 8****:** View of a microscope (28) located on the bottom inside the bioreactor (1), and formed by a lamp (29), a filter holder (30), a slide diffuser (31), a iris (32), focus adjustment (33), a condenser (34), the sample holder support (35), lens (36), a digital camera (37) and a set of three axes - Y (38), X (39) and Z (40) -.

### Mode for invention

The modular equipment for processing and storage of cell cultures can be performed and arranged as follows:

The equipment consists of several basic modular spaces or areas: a bioreactor (1), a load beds area and discharge of beds used (2), a supply zone of culture and reagents (3), an area of pumping and centrifugation (4), and an area of data processing computer containing a microprocessor - the central processing unit (CPU) - and storage of wastes and scraps (5).

Arranged inside the bioreactor (1), can be used one or more modular parts, such as chain columns of cells - physic units that contains the cultivations or cells cultures on beds - (6), centrifuge (27), and a microscope (28) according to the dimensions of equipment, workspace where the equipment is or needs work.

Save the bioreactor (1) and centrifugation (4), the load beds area and discharge of beds used (2), the area of culture and supply of reagents (3), containing gates that can be open or closed in accordance with operational needs.

Equipment operation is initiated by the arrangement of beds (12) - Fig.4 in the inbox of beds to use (13). An engine or other method of thrust pushes the bed to use (12) placing it on the transport support (17) that moves through a transport guide (16) heading towards the chain of cells (6) located within the bioreactor (1).

The interior of the bioreactor (1), and closed chamber, available-Fig.3 one or several chain columns of cells (6) with his foot column (11) and link chain or strap cells (10.) for transmitting the movement of the chain or strap cells can use a motor on one of the rotors whether either the top or bottom of the chain (8) or (9) that transmit the movement to all chain or other transmission method already known in the case of the cells were joined together in solidarity, they might act as a method of transmission.

Note the inclusion of a damping system of the cells (7) that reduces the vibration of the beds (12). Each cell (7) may contain one or more beds (12) plate-type, platelet, or pouches - by way of example, the most common commercial and Autoflask, OptiCell, Petak G2, etc ... - the dimensions and stacking slots each of which vary depending on the type of tables.

The empty bed (12) located in a slot of the cells (7) moves to the position where the piece is injection or extraction and sterilization (18), located at the bottom of the inside of the bioreactor (1) or accessible place from the chain of cells (6). Once the bed (12) is placed against the driver of beds (24), the latter through a bed driving lever (25) and a mobile base of the piece driving beds (26) horizontal scroll executed maneuvers or out of bed to the injection of fluids.

While the bed (12) is hosted on a piece of injection and sterilization (18), it can be processed with a given cell culture, reagent or nutrient medium by introducing compressed air through a chamber of pre-injection ( 21) and culture fluid through an injection fluid conduit (20). The liquid nutrient culture or injected is stored in the supply of culture (3) and are directed to the injection module through peristaltic pumps or any other method of discharge in the pumping and centrifugation (4).

Within a pre-injection chamber (21) performed the addition and mixing of fluids or culture culture, which are injected into the bed (12) through the mouth of the nozzle (22). This guaranteed performed under aseptic conditions by a equipment of sterilization (23) of electromagnetic type - magnetron - or, alternatively, by thermal, radiation or otherwise.

Once again the culture medium or liquid has been distributed within the bed (12), it returns to be housed in the same position within the cell (7) using a system similar to that used for the input of the module injection (18)

This procedure is repeated pre-programmed to gradually introduce and processing each and every task in the chain of cells (6)

The chain column of cells (6) is thus in constant tumbling motion and without any vibration or shock, stopping for operational processing of crops and environmental conditions aseptic, stable and uniform within the bioreactor (1 ), either to different degrees of temperature or pressure that would require each crop processing. So it is controlled at all times the position of each of the beds (12) in cells (7) that is stored in the processing unit.

Later, we proceed to the discharge of beds with cultivation used (2) by subtracting the beds for the piece driving beds (24), and their placement in an output tray (14) through a transport support (17) moving along a transport guide (16) to said output tray. This process occurs through a lever that is deposited beds in the output tray (14) which is accessible from the opening gap (15) which may be available to the user directly.

One of the tasks in the multiple may be made within the cell culture is centrifugation of the beds (12) for the separation of subjects in these

The piece centrifuge spins at 2000 rpm for more than a time determined by the user from the programming of control tasks. A system as occurs with the driver of beds (24) to injection module (18), remove the bed of the chain of cells (6) and leaves the previous stay in the queue entry to the centrifuge module. A linear drive him into the centrifuge module (27).

For the extraction bed (12), a flexible system pushes the bed (12) from the centrifuge module (27) to the previous stay in the queue. Bedding (12) returns to the chain of cells (6) by means of a flange mounted on another drive linear.

This module of the centrifuge (27) acts as a trapping mechanism activated by the centrifugal force due to rotation thereof. It also allows the spin of one or more beds simultaneously. As an intermediate step between the centrifuge module (27) and the chain of cells (6) can be provided a cultured beds storage buffer (12) in the input queue and output.

For distributed monitoring of the crop in the bed (12) can have one or more microscopic module (28) that captures images of the same to perform calculations and cell counts and high resolution photographs of the samples of cell culture . These and other possible processes that may be made by the microscope are defined by the user, depending on what you want to monitor and / or calculate, and it can choose the scanning resolution.

The microscope is mounted on three linear axes - X, Y, Z - (39, 38 and 40) that is controlled by the central processing unit (5). The positions of the X and Y can be consecutive or not, but the positioning of the Z axis must be the last. After placing the cursor on the bed of culture (12) takes a first look at X and Y determine the focal point and the position of the target Z, all controlled by the central processing unit (5). Z axis motion in addition to acting approach has a computerized image processing. Thus in every shot you know the number of dead or living cells.

Bedding (12) is extracted from the chain of cells (6) by a system equal to the impeller of beds (24) of pre-injection module (18), and places it on a cookie scan, checking the ID code bed (12). Once confirmed that begins monitoring is controlled by the central processing unit - CPU - (5).

All equipment is controlled and monitored by focusing processing unit (5). This system works with a client-server configuration that lets you control all the parameters of the equipment (environmental conditions within the bioreactor bed position within the cell, to carry out tasks, times, etc.). Whether it be physically in front of the equipment through its control screen, like from any other computer connected to the network.

## Claims

1. Method and modular equipment for processing and storage of cell cultures **characterised by** an arrangement of beds (12) in the inbox of beds to use (13). A motor drives the site to use (12) placing it on the transport support (17) that moves through a transport guide (16) heading towards the chain of cells (6) located within the bioreactor (1). Inside the bioreactor (1) there is a chain column of cells (6) with your foot column (11) and chain (10). For the transmission of movement of the chain using a rotor-motor (8) at the top of the chain will transmit the movement to all the chain to the spinning rotor (9) conducting a continuous tumbling motion. Each cell (7) consists of a vibration damper may contain one or more beds (12). The empty bed (12) located in a slot of the cells (7) moves to the position where the piece of injection and sterilization (18), located at the bottom of the inside of the bioreactor (1) or any place accessible from the chain of cells (6). Once the bed (12) faces the driver of beds (24), this, through a lever driving beds (25) and a mobile base of the piece driving beds (26) horizontal scroll maneuvers or out of bed to the injection of fluids. While the bed (12) is hosted on a piece of injection and sterilization (18), it is processed with a given cell culture, reagent or nutrient medium by introducing compressed air through a pre-injection chamber (21) and the provision of a liquid culture through a duct injection fluid (20). The injected liquid nutrient culture or are stored in the supply of culture (3) and are directed to the injection module through peristaltic pumps in the pumping and centrifugation (4). Within this pre-injection chamber (21) performed the above addition and mixing of fluids or culture culture, which are injected into the bed (12) through the mouth of the nozzle (22). All running under aseptic conditions through a equipment sterilization (23) of electromagnetic type - magnetron -. Once the fluid or medium has been distributed within the bed (12), it returns to be housed in the same position within the cell (7) using a system similar to used for the input of the injection module (18). Later, we proceed to discharge of bed used (2) by removing the beds by the driving part (24), and their placement in an output tray (14) through a transport support (17) that moves along a transport guide (16) to said output tray. This process occurs through a lever that is deposited beds in the output tray (14) which is accessible from the opening gap (15) which may be available to the user. Following the introduction of the liquid culture medium or a piece centrifuge rotates between 1500 and 2000 rpm for the time determined by the user from programming the control tasks. A system equal and equivalent to that given to the driver of beds (24) to injection module (18), remove the bed of the chain of cells (6) and leaves the room prior to the centrifuge module. A linear drive him into the centrifuge module (27). For the extraction bed (12), a flexible system pushes the bed (12) from the centrifuge module (27) to the previous stay. Bedding (12) returns to the chain of cells (6) by means of a flange mounted on another drive linear. This module of the centrifuge (27) acts as a trapping mechanism activated by centrifugal force itself, due to the rotation thereof. For distributed monitoring of the crop in the bed (12) can have a microscopic module (28) that captures images of the same to perform calculations and cell counts and high resolution photographs of the samples of cell culture. These and other possible processes that may be made by the microscope are defined by the user, depending on what you want to monitor and / or calculating, choosing likewise the scanning resolution. The microscope is mounted on three linear axes - X , Y, Z - (39, 38 and 40) that are controlled not interpolated. The positions of the X and Y can be consecutive or not, but the positioning of the Z axis must be the last. After placing the cursor on the bed of culture (12) takes a first look at X and Y determine the focal point and indicating the position of the target Z, all controlled by the central processing unit (5). The Z axis movement of focus in addition to acting has a computerized image recognition of the familiar. Bedding (12) is removed from the chain of cells (6) by a system equal to the impeller of beds (24) of pre-injection module (18), and places it on a cookie scan, checking the ID code bed (12). Once confirmed, begin monitoring that is controlled by the central processing unit.

2. Method and equipment for processing and modular storage cell culture according to claim 1 **characterised by** a bioreactor (1), a load beds area and discharge of beds used (2), an area of supply of cultures and reagents (3), an area of pumping and centrifugation (4), and a processing area of computer data containing a microprocessor - the central processing unit (CPU) - and storage of waste and scrap (5 ).

3. Method and modular equipment for processing and storage of cell cultures according to claims 1 and 2 **characterised** wherein the zone of pumping and centrifugation (4), the load beds area and discharge of beds used (2) and the area of supply of cultures and reagents (3), containing gates that can be opened or closed according to operational needs.

4. Method and modular equipment for processing and storage of cell cultures according to claims 1 and 2 **characterised by** the chain that links with cells (10) is replaced by a belt with cells or cells themselves joined together in solidarity to act as a means of transmission.

5. Method and modular equipment for processing and storage of cell cultures according to claims 1 and 2 **characterised in that** for transmitting the movement of the spine chain of cells (6) the rotor-motor (8) provided in the bottom of the column, in the vicinity of the chain of cells (11), while the spinning rotor (9) is set on top.

6. Method and modular equipment for processing and storage of cell cultures according to claims 1 and 2 **characterized in that** between the centrifuge module (27) and the chain of cells (6) there is a buffer storage beds (12).

7. Method and modular equipment for processing and storage of cell cultures according to claims 1 and 2, **characterized in that** inside the bioreactor (1) can be used one or more modular pieces of chain columns of cells (6), a centrifuge (27), and microscopes (28).

8. Method and modular equipment for processing and storage of cell cultures according to claims 1 and 2 **characterised** wherein the sterilization equipment (23) uses thermal means and / or radiation.

9. Method and modular equipment for processing and storage of cell cultures according to claims 1 and 2, **characterized by** it has a client-server configuration telematics control screen connected to the data processing unit and / or external drives.
